# EUROPÄISCHE PATENTSCHRIFT

(11) **EP 2 519 153 B1**
(45) Veröffentlichungstag und Bekanntmachung des Hinweises auf die Patenterteilung: **11.02.2015**
(21) Anmeldenummer: 10805788.6
(22) Anmeldetag: 24.12.2010
(51) Int. Cl.: A61B 5/22, A61B 5/00

(54) **VORRICHTUNG UND VERFAHREN ZUM ERFASSEN UND MESSEN VON SCHMERZEN**
DEVICE AND METHOD FOR DETECTING AND MEASURING PAIN
DISPOSITIF ET PROCÉDÉ PERMETTANT DE CONSIGNER ET DE MESURER UNE DOULEUR

(30) Priorität: 07.02.2010 WO PCT/EP2010/051455; 28.12.2009 WO PCT/EP2009/067955
(43) Veröffentlichungstag der Anmeldung: 07.11.2012
(73) Patentinhaber: MSYS AG, 8008 Zürich (CH)
(72) Erfinder: SCHAFFNER, Nils, CH-8006 Zürich (CH); FOLKERS, Gerd, CH-8049 Zürich (CH); BRINKHAUS, Bernhard, CH-8955 Oetwil a. d. Limmat (CH); SCHUURMANS STEKHOVEN, Marco, CH-8008 Zürich (CH)
(74) Vertreter: Dr. Graf & Partner AG
(86) Internationale Anmeldenummer: PCT/EP2010/070736
(87) Internationale Veröffentlichungsnummer: WO 2011/080237

(56) Entgegenhaltungen:
- WO-A1-2008/028572
- WO-A2-01/87143
- WO-A2-2009/052100
- DE-U1-202008 004 130
- GB-A- 2 027 901
- GB-A- 2 434 650
- US-A- 2 989 357
- US-A- 5 119 831
- US-A1- 2004 243 021
- US-A1- 2006 063 647

## Beschreibung

Die Erfindung betrifft eine Vorrichtung zum Erfassen und Messen von Schmerzen gemäss Anspruch 1. Die Erfindung betrifft weiter eine Verwendung der Vorrichtung gemäss Anspruch 15.

### Stand der Technik

Schmerzen gehören zu den häufigsten Gründen einen Arzt aufzusuchen. Auf die Frage des Arztes wie stark denn die Schmerzen sind, findet der Patient oft keine klar verständliche Antwort. Entweder findet er nicht die richtigen Worte oder der Arzt versteht die benutzen Worte nicht. Dies liegt unter anderem daran, dass der Schmerz eine subjektive Sinnesempfindung ist, die sensorische, kognitive und emotionale Aspekte beinhaltet. Eine akkurate Schmerzmessung ist jedoch sehr wichtig, z.B. für die Diagnose, beim Anpassen der medikamentösen Schmerztherapie oder in der Schmerzmittelforschung.
Eine Vielzahl an Methoden, um Schmerzen zu erfassen wurde bis heute entwickelt. Die meisten lassen sich in zwei Kategorien einteilen: Eindimensionale Schmerzskalen und multidimensionale Schmerzfragebögen. Die wichtigste eindimensionale Skala ist die "Visuelle Analog Skala" (VAS). Sie besteht aus einer 10 cm langen Linie, deren linkes Ende mit "kein Schmerz" und deren Rechtes mit "stärkster vorstellbarer Schmerz" bezeichnet ist. Der Patient markiert mit einem Strich den Punkt auf der Linie, der seinem Schmerz entspricht. Eng verwandt mit der VAS ist die NRS (Numerische Rating Skala), wobei die Auswahl einer Zahl von 0-10 zur Schmerzdarstellung mündlich mitgeteilt wird. Die VAS wird am meisten verwendet, da sie sehr unkompliziert ist und keine grossen Erklärungen benötigt. Das Problem solcher Skalen ist jedoch, dass man als Ausfüllender generell zur Mitte tendiert und ungern die Extreme markiert. Auch bei einer mehrfachen Benutzung der VAS tendieren die Anwender bei unterschiedlicher Schmerzintensität zu einer immer näher aneinander liegenden Bewertung (Convergenz). Dies hat zur Folge, dass der Arzt mit dieser Erfassungsmethode den Eindruck gewinnt, es lägen nur kleine Änderungen vor.
Der wichtigste Schmerzfragebogen ist der McGill Pain Questionnaire (MPQ). Der Patient erhält eine grosse Auswahl an Adjektiven von denen er diejenigen ankreuzt, die seinem Schmerz entsprechen. Die Adjektive sind in drei Klassen eingeteilt: Sensorisch, affektiv und evaluativ. So werden zwar mehrere Aspekte des Schmerzes erfasst, dafür dauert das Ausfüllen lange und der Patient muss die Adjektive auch alle verstehen respektive kennen, um den MPQ richtig auszufüllen.
Es gibt verschiedene technische Geräte, beispielsweise die in der Druckschrift EP 0874587 B1 offenbarte Vorrichtung, um Schmerzen zu erfassen. Jedoch sind dies meisten computerisierte Varianten der bekannten Skalen VAS oder Fragebögen MPQ inklusive deren Nachteile. Des Weiteren gibt es verschiedene Geräte zum Applizieren von Schmerzreizen für die Schmerzschwellen- und Schmerztoleranzmessung, z.B. die in der Druckschrift WO 2004/103230 offenbarte Vorrichtung.

Die Druckschrift WO 2009/052100 offenbart eine weitere Vorrichtung zum Messen von Schmerzen. Diese Vorrichtung weist den Nachteil auf, dass der Schmerz nur äusserst ungenau messbar ist, und dass die Vorrichtung nur geeignet ist um Schmerzen im Darm zu messen.

### Darstellung der Erfindung

Aufgabe der vorliegenden Erfindung ist es eine vorteilhaftere Vorrichtung zur Erfassung von Schmerzen zu bilden.

Diese Aufgabe wird gelöst mit einer Vorrichtung umfassend die Merkmale von Anspruch 1. Die Unteransprüche 2 bis 14 beziehen sich auf weitere, vorteilhafte Ausgestaltungen. Diese Aufgabe wird weiter gelöst mit einer Verwendung der Vorrichtung gemäss Anspruch 15.

Die Aufgabe wird insbesondere gelöst mit einer Vorrichtung zum Erfassen und Messen von Schmerzen eines Menschen, umfassend einen Druck- oder Kraftsensor, umfassend einen Hohlkörper mit einer Aussenhülle und einem Innenraum, wobei die Aussenhülle den Innenraum zumindest teilweise begrenzt, sowie umfassend eine Elektronikeinheit zum Erfassen des Signals des Druck- oder Kraftsensors, wobei die Aussenhülle des Hohlkörpers derart ausgestaltet ist, dass diese von einer Hand zumindest teilweise umschliessbar ist, und wobei der Innenraum des Hohlkörpers mit einem elastischen Material oder einem Fluid gefüllt ist, und wobei der Druck- oder Kraftsensor derart angeordnet ist, dass der Druck des elastischen Materials oder des Fluides messbar ist, wobei die Aussenhülle hohlzylinderförmig ausgestaltet ist, die Aussenhülle mit einem oberen Endteil sowie einem unteren Endteil verbunden ist, das obere Endteil, das untere Endteil sowie die Aussenhülle den Innenraum begrenzen, die Aussenhülle elastisch ausgestaltet ist, und das obere und untere Endteil starr ausgestaltet ist.

Die erfindungsgemässe Vorrichtung bezieht sich auf das Gebiet der Schmerzmessung und deren Erfassungssysteme. Die Ergebnisse solcher Schmerzmessungen dienen zur Diagnose, Forschung und zur Findung von Linderungsmöglichkeiten. Die erfindungsgemässe Vorrichtung ermöglicht es Schmerzen zu erfassen und vorzugsweise auch die Schmerzen gewissen Schmerzniveaus zuzuordnen. In einer vorteilhaften Ausgestaltung ermöglicht es die erfindungsgemässe Vorrichtung auch Schmerzen zu generieren. Ein wesentlicher Vorteil der erfindungsgemässen Vorrichtung ist darin zu sehen, dass diese es ermöglicht Schmerzen objektiv zu messen und deren Höhe vorzugsweise in gewisse Niveaus zu ordnen. Ein Mensch weist die Eigenschaft auf, dass dieser bei Schmerzen, insbesondere bei starken Schmerzen, die Hand reflexartig zusammenzieht, wobei sich die Handinnenfläche bis zur Ausbildung einer Faust zusammenziehen kann. Um dieses reflexartige Verhalten von Menschen zu nutzen ist die erfindungsgemässe Vorrichtung derart ausgestaltet, dass diese einen Hohlkörper aufweist, der von einer Hand zumindest teilweise umschliessbar ist. In einer besonders vorteilhaften Ausgestaltung ist der Hohlkörper rohrförmig ausgestaltet, insbesondere als geradlinig verlaufendes Rohr. Ein derartig ausgestalteter Hohlkörper liegt gut in der Hand und nutzt die natürliche, reflexartige Bewegung des Menschen zur Messung von Schmerzen, in dem die Hand den Hohlkörper mit zunehmendem Schmerz auf natürliche weise stärker und kraftvoller hält. Die Fläche des Hohlkörpers, welche zur Anlage an der Hand bestimmt ist, sollte vorzugsweise formstabil oder im Wesentlichen formstabil ausgestaltet sein, was den Vorteil ergibt, dass sich dessen Form unabhängig von der angreifenden Kraft der Hand nicht oder nur sehr geringfügig ändert. Dies ermöglicht die von der Hand bewirkten Kraft reproduzierbar und genau zu messen. Anders ausgedrückt, würde sich die Form des Hohlkörpers auf Grund der von der Hand auf den Hohlkörper bewirkten Kraft ausgeprägt verändern, was zum Beispiel bei einem mit Luft gefüllten Gummibalg der Fall wäre, so wäre der empfundene Schmerz nicht mehr über die von der Hand bewirkten Kraft messbar, da der Gummibalg bei zunehmender Kraft nachgibt und dessen Form verändern, sodass es schwierig oder nicht mehr möglich ist den empfundenen Schmerz über die Kraft vorzugsweise linear auszudrücken, insbesondere wenn die Hand bereits zur Faust geballt ist. Es kann sich als vorteilhaft erweisen die Oberfläche des Hohlkörpers derart zu gestalten, dass sich an der Handfläche, welche an der erfindungsgemässen Vorrichtung anliegt, keine ausgeprägten und eventuell sogar schmerzhaften Druckstellen ausbilden. Solche Druckstellen sind unangenehm und könnten sogar die Messung verfälschen. In einer vorteilhaften Ausgestaltung weist die Oberfläche des Hohlkörpers, welche zur Anlagen an der Hand bestimmt ist, eine gewisse Elastizität auf, um an der anliegenden Hand derartige Druck- oder Schmerzpunkte zu vermeiden. Die erfindungsgemässe Vorrichtung ermöglicht es die von der Hand bewirkte Druck- oder Presskraft besonders genau zu messen. Der Innenraum des Hohlkörpers ist dabei vorzugsweise mit einem elastischen, nicht gasförmigen Material oder einem nicht gasförmigen Fluid gefüllt, wobei ein Druck- oder Kraftsensor den Druck des elastischen Materials oder des Fluides misst, welcher durch die an der Vorrichtung angreifende Hand erzeugt wurde. Die erfindungsgemässe Vorrichtung erlaubt es die Stärke des empfundenen Schmerz bei einer individuellen Person zuverlässig und auch reproduzierbar zu messen.

Als elastisches Material ist insbesondere ein Material wie Silikon, Kautschuk, Gummi oder ein Gel geeignet. Solche Materialien sind üblicherweise nicht fliessfähig. Der Innenraum kann jedoch auch mit einem nicht gasförmigen Fluid wie eine Flüssigkeit oder ein Gel gefüllt sein, um die an der Aussenhülle des Hohlköpers angreifende Kraft zuverlässig zum Druck- oder Kraftsensor zu übertragen. Die erfindungsgemässe Vorrichtung, deren Innenraum mit einem derartigen Material gefüllt ist, weist den Vorteil auf, dass die angreifende Kraft hysteresefrei oder mit sehr geringer Hysterese auf den Druck- oder Kraftsensor übertragbar ist, was zur Folge hat, dass die angreifende Kraft und auch geringe Veränderungen der Kraft sehr genau gemessen werden kann, sodass beispielsweise auch geringe Veränderungen der Kraft und insbesondere auch eine abnehmende Kraft, insbesondere eine geringfügig abnehmende Kraft, sehr genau gemessen werden kann. Die erfindungsgemässe Vorrichtung ermöglicht somit sowohl vorzugsweise die absolute Kraft als auch Kraftveränderungen, welche durch die Hand bewirkt werden, genau, reproduzierbar und insbesondere mit geringer Hysterese oder sogar hysteresefrei zu messen.

Die erfindungsgemässe Vorrichtung weist insbesondere den Vorteil auf, dass die Schmerzen und vorzugsweise auch deren Zustände genau erfassbar sind, und dass die Messungen der Schmerzen objektiv reproduzierbar sind.

Die Erfindung wird nachfolgend an Hand von Ausführungsbeispielen beschrieben.

### Kurze Beschreibung der Zeichnungen

Die zur Erläuterung der Ausführungsbeispiele verwendeten Zeichnungen zeigen:
- Fig. 1: schematisch einen Längsschnitt durch ein Ausführungsbeispiel einer Vorrichtung zum Erfassen von Schmerzen;
- Fig. 2: schematisch eine Seitenansicht eines weiteren Ausführungsbeispiels einer Vorrichtung zum Erfassen von Schmerzen;
- Fig. 3: schematisch eine Seitenansicht eines weiteren Ausführungsbeispiels einer Vorrichtung zum Erfassen von Schmerzen;
- Fig. 4: schematisch einen Schnitt durch ein Gehäuse mit integriertem Druck- oder Kraftsensor;
- Fig. 5: schematisch eine Seitenansicht eines weiteren Ausführungsbeispiels einer Vorrichtung zum Erfassen von Schmerzen;
- Fig. 6: die Ergebnisse einer bekannten Vorrichtung zum Messen von Schmerzen;
- Fig. 7: die Ergebnisse der erfindungsgemässen Vorrichtung zum Messen von Schmerzen;
- Fig. 8: ein Beispiel einer Eichung der erfindungsgemässen Vorrichtung;
- Fig. 9: schematisch ein weiteres Ausführungsbeispiel einer Vorrichtung zum Erfassen von Schmerzen;
- Fig. 10: schematisch ein weiteres Ausführungsbeispiel einer Vorrichtung zum Erfassen von Schmerzen;
- Fig. 11: einen Längsschnitt durch ein weiteres Ausführungsbeispiel einer Vorrichtung zum Erfassen von Schmerzen entlang der Schnittlinie B-B;
- Fig. 12: einen Querschnitt entlang der Linie A-A durch die in Figur 11 dargestellte Vorrichtung.
- Fig. 13: einen Längsschnitt durch ein weiteres Ausführungsbeispiel einer Vorrichtung zum Erfassen von Schmerzen entlang der Schnittlinie D-D;
- Fig. 14: einen Querschnitt entlang der Linie C-C durch die in Figur 13 dargestellte Vorrichtung.

Grundsätzlich sind in den Zeichnungen gleiche Teile mit gleichen Bezugszeichen versehen.

### Wege zur Ausführung der Erfindung

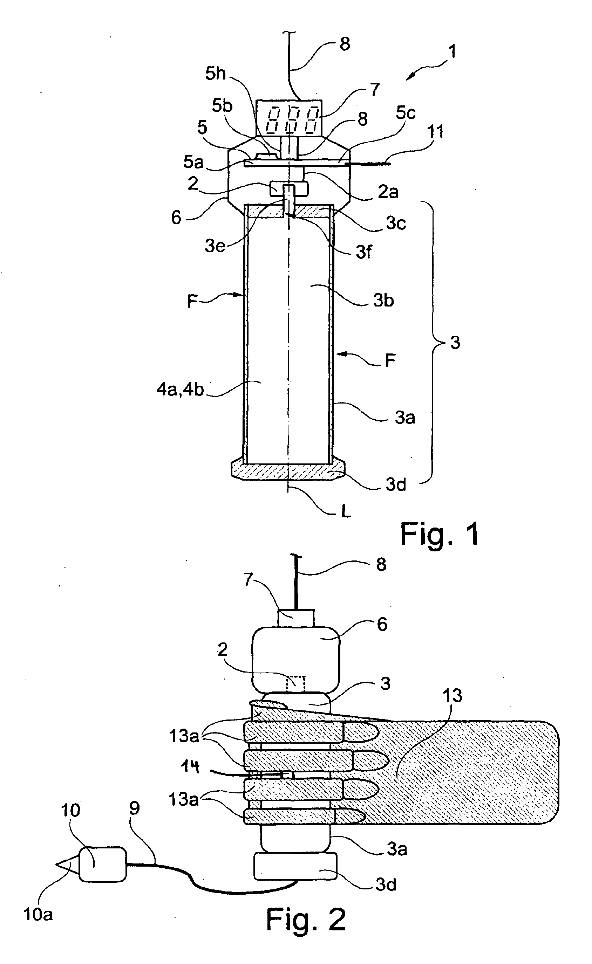

Fig. 1 zeigt in einem schematischen Längsschnitt eine Vorrichtung 1 zum Erfassen von Schmerzen. Die Vorrichtung 1 zum Erfassen von Schmerzen umfasst einen Hohlkörper 3 mit einer elastischen Aussenhülle 3a, welche einen Innenraum 3b wie dargestellt zumindest teilweise begrenzt. Die elastische Aussenhülle 3a könnte beispielsweise aus Silikon, Gummi oder Kautschuk gefertigt sein. Im dargestellten Ausführungsbeispiel ist die Aussenhülle 3a mit einem oberen Endteil 3c sowie einem unteren Endteil 3d verbunden, wobei das obere Endteil 3c, das untere Endteil 3d sowie die Aussenhülle 3a den Innenraum 3b begrenzen, sodass der Innenraum 3b vorzugsweise Fluid dicht ist. Erfindungsgemäss ist das obere und untere Endteil 3c, 3d starr ausgestaltet und ist beispielsweise aus einem Kunststoff oder aus Metall gefertigt. Im dargestellten Ausführungsbeispiel ist die Aussenhülle 3a hohlzylinderförmig ausgestaltet, wobei das obere und das untere Endteil 3c,3d kreisscheibenförmig ausgestaltet sind, sodass der Hohlkörper 3 eine Längsachse L aufweist, welche in einer vorteilhaften Ausgestaltung bezüglich dem Hohlkörper 3 zudem eine Symmetrieachse ausbildet. Das obere Endteil 3c weist in der Mitte eine kreisförmige Öffnung 3f auf, ausgehend von welcher sich eine rohrförmige Verlängerung 3e in Verlaufsrichtung der Längsachse L erstreckt. Am Ende der rohrförmigen Verlängerung 3e ist ein Druck- oder Kraftsensor 2 angeordnet. Der Innenraum 3b des Hohlkörpers 3 sowie die rohrförmige Verlängerung 3e sind vollständig mit einem nicht sichtbar dargestellten elastischen Material 4a oder einem Fluid 4b gefüllt. Besonders vorteilhaft ist der Innenraum 3b mit einem elastischen, nicht gasförmigen Material 4a oder ein nicht gasförmigen Fluid 4b gefüllt. Dies hat zur Folge, dass beispielsweise eine auf die Aussenhülle 3a ausgeübte Druckkraft F vom Druck- oder Kraftsensor 2 erfasst werden kann, da die auf Grund der Druckkraft F im Innenraum 3b bewirkte Druckerhöhung vom elastischen Material 4a oder vom Fluid 4b auf den Druck- oder Kraftsensor 2 übertragen wird. Als Fluid ist ein nicht gasförmiges Fluid wie eine Flüssigkeit oder ein Gel geeignet. In einer bevorzugten Ausgestaltung weist das elastische Material 4a eine geringere Elastizität auf als die Aussenhülle 3a, damit sich die Aussenhülle 3a angenehm elastisch anfühlt, und damit die Kraft F besonders gut in das elastische Material 4a eingeleitet werden kann. Wie in Figur 1 dargestellt bildet die elastische Aussenhülle 3a die Trennung zwischen Innenraum 3b vom Aussenraum entlang der Längsrichtung L, beginnend beim oberen Endteil 3c und endend beim unteren Endteil 3d. Der Durchmesser des Hohlkörpers 3 ist derart gewählt, dass die elastische Aussenhülle 3a von einer Hand 13 zumindest teilweise umfasst werden kann, sodass die Hand 13 angenehm auf die elastische Aussenhülle 3a aufgelegt werden kann, und die Hand 13 die elastische Aussenhülle 3a in Umfangsrichtung teilweise oder vollständig umschliesst. Beim Zusammendrücken der Hand 13 wird eine Kraft F auf die elastische Aussenhülle 3a bewirkt, was zur Folge hat, dass der Druck des sich im Innenraum 3b befindlichen elastischen Materials 4a oder des Fluides 4b ansteigt. Der Druck- oder Kraftsensor 2 kann somit den Druck im Innenraum 3b beispielsweise in mbar oder Newton messen. In einer besonders vorteilhaften Ausgestaltung ist zumindest derjenige Teil der Oberfläche der erfindungsgemässen Vorrichtung 1, welcher während der Messung von der Hand gehalten wird, als elastische Aussenhülle 3a ausgestaltet, wobei die elastische Aussenhülle 3a wie in Figur 1 dargestellt, unmittelbar den Innenraum 3b vom Aussenraum begrenzt. Wenn die Hand während des Zusammendrückens beziehungsweise während des Messens des Schmerzes ausschliesslich die elastische Aussenhülle 3a berühren, so ergibt dies den Vorteil, dass die Hand an keinerlei Druckstellen anliegt, was die Schmerzmessung verfälschen könnte. Würde die Hand beim Zusammendrücken beispielsweise teilweise am Gehäuse 6 und/oder am oberen Endteil 3c und/oder am unteren Endteil 3d anliegen, so würde der Patient beim Zusammendrücken reflexartig die Druckkraft verringern, um eventuell an der Hand auftretende Schmerzen und/oder unangenehme Gefühle zu vermeiden. Die derart ausgestalteten und angeordneten elastischen Aussenhülle 3a, in Kombination mit dem sich im Innenraum 3b befindlichen elastischen Materials 4a oder des Fluides 4b, und im Kombination mit dem Druck- oder Kraftsensor 2, welcher den Druck im Innenraum 3b erfasst, ermöglicht es die Anpresskraft der Hand reproduzierbar und genau zu messen, sodass der subjektiv empfundene Schmerz, welcher von einem Patienten über die Anpresskraft der Hand ausgedrückt wird, gemessen werden kann. Untersuchungen mit Patienten haben überraschenderweise gezeigt, dass eine derartige Vorrichtung geeignet ist, um subjektiv empfundene Schmerzen über die Anpresskraft der Hand reproduzierbar zu messen.

Die in Figur 1 dargestellte Vorrichtung 1 umfasst zudem ein Gehäuse 6, welches einerseits mit dem oberen Endteil 3c befestigt ist, und welches andererseits mit einer Anzeigevorrichtung 7 befestigt ist. Innerhalb des Gehäuses 6 ist eine Elektronikeinheit 5 angeordnet, welche im dargestellten Ausführungsbeispiel eine Leiterplatte 5a, elektronische Bausteine 5b, sowie eine Lese- und Schreibvorrichtung 5c für eine Speicherkarte 11 umfasst. Die Speicherkarte 11 ist vorzugsweise, wie in Figur 1 dargestellt, von Aussen in die Elektronikeinheit 5 einschiebbar und vorzugsweise auch auswechselbar. Die Elektronikeinheit 5 ist über Signalleitungen mit der Anzeigevorrichtung 7 verbunden. Zudem ist die Elektronikeinheit 5 über eine Signalleitung 2b mit dem Druck- oder Kraftsensor 2 verbunden. Zudem ist die Elektronikeinheit 5 über ein Kabel 8 mit einer ausserhalb der Vorrichtung 1 angeordneten Vorrichtung verbunden. Das Kabel 8 kann beispielsweise zur Energieversorgung und/oder zur Übertragung von Signalen oder Daten dienen. Die elektronischen Bausteine 5b könnten zudem eine nicht dargestellte Auswertevorrichtung 5f und/oder eine akustische Ausgabevorrichtung 5g umfassen.

In einer weiteren möglichen Ausführungsform könnten das obere und untere Endteil 3c,3d auch gegenseitig direkt mit einander verbunden sein, zum Beispiel durch eine im Innenraum 3b in Richtung der Längsachse L verlaufende Verbindungsstange, welche die beiden Endteile 3c,3d gegenseitig fest verbindet.

Figur 2 zeigt ein weiteres Ausführungsbeispiel einer Vorrichtung 1 zum Erfassen und Messen von Schmerzen in einer Seitenansicht. Die Vorrichtung 1 umfasst einen Hohlkörper 3 mit einer elastischen Aussenhülle 3a, wobei der Hohlkörper 3, gleich wie in Figur 1 dargestellt, oben und unten von einem oberen Endteil sowie einem unteren Endteil 3d begrenzt ist. Das obere Endteil sowie der gestrichelt dargestellte Druck- oder Kraftsensor 2 sind innerhalb des Gehäuses 6 angeordnet. Am Gehäuse 6 ist zudem eine Anzeige 7 angeordnet. Zudem ist wie in Figur 1 beschrieben ein Verbindungskabel 8 vorgesehen. Figur 2 zeigt zudem schematisch eine Hand 13 mit Fingern 13a, wobei die Finge 13a die elastische Aussenhülle 3a umschliessen. Die von der Hand 13 auf die elastische Aussenhülle 3a bewirkte Kraft kann mit dem Druck- oder Kraftsensor 2 erfasst werden.

Die erfindungsgemässe Vorrichtung 1 kann in einem Ausführungsbeispiel zudem einen Schmerzaktuator 10 umfassen, der beispielsweise über eine elektrische Leitung 9 mit der Vorrichtung 1 verbunden ist. Der Schmerzaktuator 10 umfasst eine Vorrichtung 10a um einen kontrollierten Schmerz zu erzeugen, beispielsweise eine Nadel oder eine erwärmbare beziehungsweise heizbare Kontaktfläche wie eine heisse Spitze.

Die erfindungsgemässe Vorrichtung 1 kann zudem in einem weiteren Ausführungsbeispiel zumindest einen zusätzlichen Sensor 14 zum erfassen physiologischer Werte umfassen, insbesondere einen Sensor zum Erfassen der Hautleitfähigkeit, oder einen Sensor zur Erfassung des Pulses oder einen chemischen Sensor zum Erfassen biologischer Werte. Der zusätzliche Sensor 14 kann, wie in Figur 2 dargestellt, beispielsweise an der Oberfläche der elastischen Aussenhülle 3a oder innerhalb der elastischen Aussenhülle 3a angeordnet sein, vorzugsweise auch derart, dass ein direkter Kontakt zwischen Hand 13 und/oder Fingern 13a und dem Sensor 14 möglich ist. Der Sensor 14 könnte jedoch auch an einer anderen Stelle der Vorrichtung 1 wie im Gehäuse 6 oder an der Oberfläche des Gehäuses 6 angeordnet sein. Das Messen physiologischer Parameter wie Hautleitfähigkeit, Blutdruck, Herzfrequenz, usw. ergibt an sich keine klare Aussage über die Stärke der Schmerzen, da diese Parameter durch emotionale Zustände wie Nervosität und Angst sowie durch Medikamente stark beeinflusst werden. Diese physiologischen Parameter können jedoch dazu dienten die Aussagen bzw. die persönliche Bewertung des Patienten zu interpretieren, um dadurch die Qualität der Schmerzmessung zu verbessern.

Die in den Figuren 1 und 2 dargestellte Vorrichtung 1 weist den Vorteil auf, dass die vom Druck- oder Kraftsensor 2 gemessene Kraft im Wesentlichen senkrecht zu der eingeleiteten Kraft F verläuft. Diese Ausgestaltung ermöglicht es die Kraft F besonders genau und insbesondere weitgehend störungsfrei zu messen.

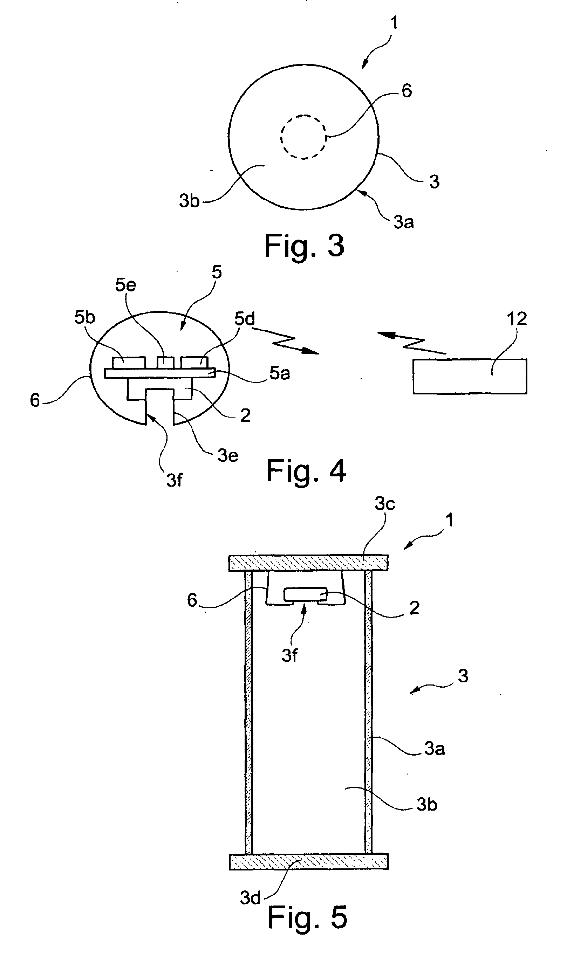

Figur 3 zeigt schematisch ein anderes Beispiel einer Vorrichtung 1 zum Erfassen und Messen von Schmerzen. Diese Vorrichtung 1 ist im dargestellten Ausführungsbeispiel kugelförmig ausgestaltet und umfasst einen Hohlkörper 3 mit einer elastischen Aussenhülle 3a und einem Innenraum 3b, wobei im Innenraum 3b ein Gehäuse 6 angeordnet ist, das in Figur 4 im Detail erläutert ist. Zudem ist der Innenraum 3b mit einem elastischen Material 4a oder einem Fluid 4b gefüllt. Die Vorrichtung 1 beziehungsweise der Hohlkörper 3 könnte auch eine andere Aussenform aufweisen und beispielsweise würfelförmig ausgestaltet sein. Auch das Gehäuse 6 kann in einer Vielzahl möglicher Formen ausgestaltet sein, und beispielsweise eine kugelförmige oder würfelförmige Aussenform aufweisen.

Figur 4 zeigt schematisch einen Schnitt durch das in Figur 3 dargestellte Gehäuse 6. Das kugelförmige Gehäuse 6 weist an der Aussenschale eine kreisförmige beziehungsweise zylinderförmige Öffnung 3f auf, von welcher aus sich eine rohrförmige Verlängerung 3e in das Innere bis zum Druck- oder Kraftsensor 2 erstreckt. Die rohrförmige Verlängerung 3e ist mit dem Druck- oder Kraftsensor 2 Fluid dicht verbunden, sodass kein Fluid in den Innenraum des Gehäuses 6 eindringen kann. Im Innenraum des Gehäuses 6 ist zudem eine Elektronikeinheit 5 angeordnet, umfassend eine Leiterplatte 5a auf welcher elektronische Bausteine 5b, eine Batterie 5d sowie eine Datenübertragungsvorrichtung 5e angeordnet sind. Die Datenübertragungsvorrichtung 5e ist vorzugsweise derart ausgestaltet, dass eine drahtlose Datenübertragung zu einer externen Sende- und Empfangsvorrichtung 12 möglich ist. Die Daten könnten beispielsweise mit Hilfe von Licht oder von elektromagnetischen Wellen übertragen werden. In einer Variante umfasst die Elektronikeinheit 5 zudem eine akustische Ausgabevorrichtung 5g, zum Beispiel einen kleinen Lautsprecher oder einen Vibrator, und/oder eine Auswertevorrichtung 5f. Die Auswertevorrichtung 5f könnte auch in der Sende- und Empfangsvorrichtung 12 angeordnet sein.

Figur 5 zeigt schematisch in einem Längsschnitt ein weiteres Ausführungsbeispiel einer Vorrichtung 1 zum Erfassen und Messen von Schmerzen. Die Vorrichtung 1 umfasst wiederum, ähnlich wie in Figur 1 dargestellt, einen Hohlkörper 3 mit einer hohlzylinderförmigen, elastischen Aussenhülle 3a welche oben und unten Fluid dicht mit einer oberen und unteren Endteil 3c,3d verbunden ist, sodass ein Fluid dichter Innenraum 3b ausgebildet ist. Im Unterschied zu der in Figur 1 dargestellten Ausführungsform ist in der in Figur 5 dargestellten Ausführungsform das Gehäuse 6 sowie der Druck- oder Kraftsensor 2 vollständig im Inneren des Innenraumes 3b angeordnet, wobei das Gehäuse 6 eine Öffnung 3f aufweist, bei welcher der Druck- oder Kraftsensor 2 angeordnet ist. Der Innenraum des Gehäuses 6 könnte auch ähnlich wie in Figur 4 dargestellt ausgestaltet sein, und insbesondere auch eine Batterie 5d und eine Datenübertragungsvorrichtung 5e umfassen.

Die erfindungsgemässe Vorrichtung 1 und insbesondere der Verlauf der Aussenhülle 3a kann in einer Vielzahl von Möglichkeiten und Formgebungen ausgestaltet werden. Die insbesondere elastische Aussenhülle 3a kann insbesondere auch der Anatomie einer Hand entsprechend ausgestaltet sein, sodass die elastische Aussenhülle 3a besonders angenehm in der Hand 13 liegt.

Eine Messung des Schmerzes mit der erfindungsgemässen Vorrichtung 1 erfolgt derart, dass ein Patient gebeten wird seine Hand an den Hohlkörper 3 anzulegen und danach durch Zudrücken der Hand intuitiv den gefühlten Schmerz auszudrücken. Die vorzugsweise rohrförmig ausgestaltete Vorrichtung 1 liegt dabei bequem in der Hand 13. Die erfindungsgemässe Vorrichtung kann, wie in den Figuren 1 und 2 dargestellt, über ein dünnes Kabel 8 mit einer nachgeordneten Vorrichtung wie einem Laptop- oder Palm-Computer verbunden werden. In einer vorteilhaften Ausgestaltung umfasst die erfindungsgemässe Vorrichtung 1 einen Massenspeicher wie einen eingebauten Logger und vorzugsweise eine Speicherkarte wie eine SD-Karte, um Daten über einen längeren Zeitabschnitt zu speichern, sodass der Patient auch während längerer Zeit selbstständig messen kann und die Daten für den Patienten unsichtbar aufgezeichnet werden. Die Vorrichtung 1 könnte eine Batterie 5d umfassen, welche beispielsweise innerhalb des Gehäuses 6 angeordnet ist. Eine derartige Vorrichtung 1 ist besonders mobil und überall einsetzbar. Die Messung erfolgt in Echtzeit und kann vorteilhafterweise über eine längere Zeitdauer durchgeführt werden. Sämtliche Daten liegen nach der Messung in digitaler Form vor. Dies ergibt den Vorteil, dass ein Arzt ohne zusätzlichen Aufwand den Verlauf der Schmerzen während der gesamten Behandlungsdauer verfolgen kann, wobei die Behandlungsdauer beispielsweise einen Tag oder eine Woche oder einen Monat dauern kann. Die erfindungsgemässe Vorrichtung weist den Vorteil auf, dass die Instruktion zum Durchführen der Schmerzmessung für den Patienten denkbar einfach sind, weil weder der Arzt noch der Patient einen grossen Wortschatz benötigen um das Bedienen der erfindungsgemässen Vorrichtung 1 sowie die damit ausgeführte Schmerzmessung zu erklären. Bisher bekannte Schmerzerfassungsverfahren erfordern, dass der Patient seinen Schmerz in Worten oder Zahlen ausdrückt, oder in Einheiten auf einer Skala ausdrückt. Die bekannten Schmerzerfassungsverfahren benötigen somit einen kognitiven Prozess, um den gefühlten Schmerz in eine Messeinheit zu übersetzen. Die erfindungsgemässe Vorrichtung weist den Vorteil auf, dass dieser kognitive Prozess nicht erforderlich ist und somit umgangen werden kann, indem der Patient intuitiv den Hohlkörper der erfindungsgemässen Vorrichtung 1 entsprechend seinem Schmerz zusammendrückt.
Damit der Arzt die Druckmessung bzw. Kraftmessung interpretieren kann, wird die erfindungsgemässe Vorrichtung 1 vor jeder Messung vorteilhafterweise kalibriert. Zum Kalibrieren wird der maximale Händedruck gemessen. In einem vorteilhaften Kalibrierverfahren werden dem Patienten zudem, zum Beispiel auf der Rückseite der freien Hand, die nicht die erfindungsgemässe Vorrichtung hält, mit Hilfe eines Schmerzaktuators 10 zum Beispiel durch erzeugen von Wärme beziehungsweise Hitze verschieden starke Schmerzreize zugeführt, die der Patient durch Drücken der erfindungsgemässen Vorrichtung 1 bewertet. Der Schmerzaktuator 10 ist vorzugsweise als eine Wärmeerzeugungsvorrichtung ausgestaltet, insbesondere als steuerbare Wärmeerzeugungsvorrichtung umfassend einen elektrischen Widerstand sowie eine Kontaktfläche, die vom elektrischen Widerstand beheizt ist. In einem vorteilhaften Kalibrierverfahren werden diese Kalibrierungsdaten zudem in anonymisierter Form gesammelt und nach Geschlecht, Alter, Krankheit und Schmerzmedikation getrennt in eine zentrale Datenbank eingespeist. Ab einer gewissen Grösse der Datenbank kann der Arzt die mit der erfindungsgemässen Vorrichtung 1 durchgeführten Messungen sowohl mit der individuellen Kalibrierung wie auch mit den Werten der Datenbank vergleichen. Dadurch wird es einem Patienten ermöglicht seinen Schmerz mit demjenigen anderer Schmerzpatienten zu vergleichen. Im Sinne einer therapeutischen Intervention (Biofeedback) kann der Patient auch versuchen gewisse Druckwerte bzw. Kraftwerte nicht zu überschreiten oder gerade eben zu erreichen.
Der Arzt kann den Patienten bitten, seinen Tagesablauf zu beschreiben und dabei mit Hilfe der erfindungsgemässen Vorrichtung 1 seinen Schmerzverlauf während dieses Tages anzugeben. Anhand dieses aufgezeichneten Schmerzverlaufs, kann der Arzt beispielsweise die Schmerzmedikation anpassen, indem er z.B. ein lang wirkendes Analgetikum einsetzt oder zu bestimmten Zeitpunkten eine schnell wirkende Tablette verordnet.

Die erfindungsgemässe Vorrichtung kann auf unterschiedlichste Weise betrieben werden. So können die Daten des Druck- oder Kraftsensors 2 beispielsweise mit einer Abtastfrequenz von vorzugsweise mindestens 10 Hz als Kurve auf dem Bildschirm eines Palm-Computers oder eines Laptops angezeigt und gespeichert werden. Auch eine Speicherung über einen intern in der Vorrichtung 1 angeordneten Logger bzw. Datenspeicher ist möglich. Zusätzlich zum Druck oder der Kraft kann auch die genaue Uhrzeit und/oder ein Markierungssignal gespeichert werden. Das Markierungssignal weist beispielsweise auf einen Zeitpunkt der Messung hin, den der Arzt später genauer anschauen will. Zum Beispiel löst er die Markierung aus, indem er auf das entsprechende Feld der Maske klickt. Nach der Messung lassen sich die Werte als Kurve oder anderen Funktionen darstellen und ausdrucken.
Zum Erfassen und Messen eines Schmerzes, den der Patient nicht mündlich äussem kann, z.B. beim Zahnarzt, lässt sich an der erfindungsgemässen Vorrichtung 1 ein Schwellenwert einstellen, bei dessen überschreiten die erfindungsgemässe Vorrichtung 1 zum Beispiel optisch oder akustisch dem Arzt mitteilt, dass der Patient gerade starke Schmerzen spürt.

Obwohl physiologische Parameter durch emotionale Zustände wie Nervosität und Angst sowie durch Medikamente stark beeinflusst werden, konnte in einer eigenen Studie gezeigt werden, dass sich Aussagen über Aufmerksamkeit und Erregtheit machen lassen. Die gleichzeitige Messung der Handwärme, der Schweisssekretion der Hand und/oder der Herzfrequenz könnten die Interpretation der mit der erfindungsgemässen Vorrichtung 1 erfassten Daten erleichtern oder vervollständigen. Daher weist die erfindungsgemässe Vorrichtung 1 in einer vorteilhaften Ausgestaltung zusätzliche Sensoren auf zum erfassen der vorhin genannten Parameter. Als besonders vorteilhaft erweist es sich diese Daten insbesondere mit den Zusatzwerten Geschlecht, Alter, Krankheit und/oder Schmerzmedikation in einer grossen Datenbank zu sammeln, was es einem Arzt ermöglicht das Verhalten seines Patienten mit einem Durchschnitt zu vergleichen.

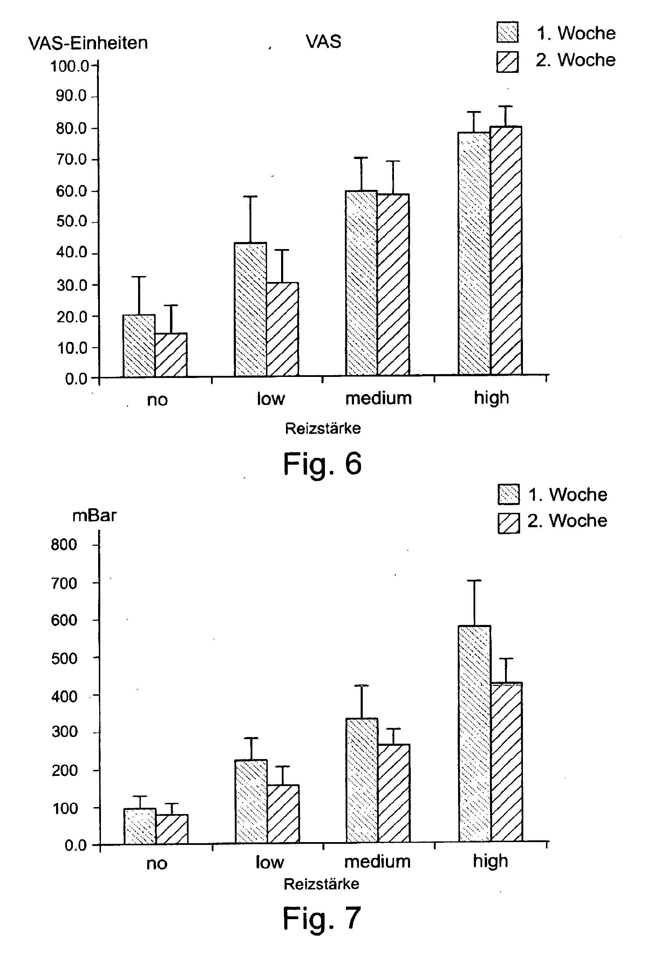

Mit der erfindungsgemässen Vorrichtung 1 wurden Studien durchgeführt. Zum einen wurden gesunden Probanden vier verschieden starke Hitzeschmerzreize auf den Unterarm appliziert. Diese mussten sie in der einen Session mit der erfindungsgemässen Vorrichtung 1 und in der anderen mit einer visuellen analogen Skala, auch als VAS bezeichnet, bewerten. Um die Wiederholbarkeit zu prüfen, wurde das Experiment exakt eine Woche später nochmals durchgeführt. Ziel dieser Studie war es zu prüfen, ob die Probanden wirklich bei den jeweils stärkeren Reizen mehr drücken, und wie die Wiederholbarkeit im Vergleich zur VAS ist. Figur 7 zeigt, dass die erfindungsgemässe Vorrichtung 1 eindeutig in der Lage ist, die verschieden starken Reize zu unterscheiden. Die in Figur 6 dargestellten Ergebnisse der VAS-Messung unterscheidet die Reize ebenfalls, kann jedoch die schwachen Reize nicht so gut auseinander halten, da die gemessenen VAS-Einheiten der schwachen Reize eine grosse Streuung aufweisen. Dies obwohl die Probanden auf Grund der für sie sichtbaren Skala sehen, wie viel sie angeben, im Gegensatz zur erfindungsgemässen Vorrichtung, 1 bei der die Probanden kein visuelles Feedback erhalten. Bezüglich der Wiederholbarkeit nach einer Woche scheint die in Figur 6 dargestellte VAS-Messung besser zu sein als die in Figur 7 dargestellte, erfindungsgemässe Messung. Da sich die Verhältnisse der verschiedenen am Probanden applizierten Reize bei der erfindungsgemässen Vorrichtung 1 jedoch nicht verändern ist es wahrscheinlich, dass die in Figur 7 dargestellten Werte der erfindungsgemässen Vorrichtung 1 eher dem wirklich gefühlten Schmerz entsprechen, und die in Figur 7 dargestellte Abnahme der Bewertung die Gewöhnung an den Reiz darstellt. Die in Figur 7 dargestellten Ergebnisse der erfindungsgemässen Vorrichtung 1 beziehungsweise des erfindungsgemässen Verfahrens weist auch eine bessere Linearität zwischen der Reizstärke und dem gemessenen Druck beziehungsweise dem gemessenen Schmerz auf, im Vergleich zu der in Figur 6 dargestellten VAS-Messung. Auch daraus ist ersichtlich, dass es die erfindungsgemässe Vorrichtung beziehungsweise das erfindungsgemässe Verfahren ermöglicht einen Schmerz genauer und reproduzierbarer zu messen.

Die Studie zeigt, dass Patienten mit Hilfe der erfindungsgemässen Vorrichtung in der Lage sind, ihre empfundenen Schmerzen beziehungsweise die auf sie ausgeübten Schmerzreize mit hoher Reproduzierbarkeit zu bewerten. Ein Vorteil der erfindungsgemässen Vorrichtung 1 ist darin zu sehen, dass diese es ermöglicht die Wirkung von verschieden starken Schmerzmitteln auf den Schmerz des Patienten sehr objektiv zu erfassen. Die Reproduzierbarkeit der VAS und NRS ist fragwürdig, da ihre Resultate dazu neigen zu konvergieren bzw. sich anzugleichen, wenn unterschiedliche Schmerzen mehrmals bewertet werden müssen. Diese Konvergenz tritt bei der erfindungsgemässen Vorrichtung 1 nicht auf. Die erfindungsgemässe Vorrichtung 1 weist den weiteren Vorteil auf, dass die Schmerzen in Echtzeit während des Auftretens der Schmerzen gemessen werden können. Der Patient kann einfach und intuitive seine Schmerzen mit Hilfe der erfindungsgemässen Vorrichtung 1 angeben beziehungsweise erfassen. Der Patient braucht dazu kein Feedback. Dies ist ein ganz wesentlicher Vorteil der erfindungsgemässen Vorrichtung im Vergleich zur VAS-Methode und anderen Methoden, die alle ein visuelles, verbales oder sonstiges Feedback benötigen. Da eine visuell (VAS) oder verbal (NRS, Numerische Rating Skala) dargestellte Zahl leicht im Gedächtnis gespeichert werden kann, wodurch sie bei späteren Darstellungen gut in Erinnerung gerufen werden kann, ist die intuitive Beurteilung der Schmerzen mit der erfindungsgemässen Vorrichtung 1, welche ohne für den Anwender merkbaren Darstellung auskommt, sehr objektiv. Die VAS-Methode weist den Nachteil auf, dass das Gedächtnis stark in die abgegebene Beurteilung der Schmerzen einbezogen wird, was zu den in der Studie herausgefundenen Fehlbeurteilungen führt.
Es hat sich gezeigt, dass auch ältere Patienten, insbesondere solche älter als 70 Jahre, keine Probleme haben die erfindungsgemässe Vorrichtung 1 anzuwenden.
Die bekannten Verfahren gemäss VAS und NRS weisen den Nachteil auf, dass diese eine sehr gute Kommunikation zwischen Patient und Arzt / Pfleger benötigen. Fehlt diese, so hat dies unvollständig aufgefüllte Patientenakten zur Folge. Die erfindungsgemässe Vorrichtung 1 sowie das entsprechende Verfahren weist den weiteren Vorteil auf, dass keine Messwerte verloren gehen. Somit können diese auch zu einem späteren Zeitpunkt ausgewertet werden. Ein weiterer Vorteil ist darin zu sehen, dass die Messwerte in digitaler Form vorliegen, und daher mit Hilfe von Kommunikationsmitteln wie Internet auch an einen anderen Ort übertragen werden können. Dadurch ist es möglich die Schmerzen eines Patienten auch von Ferne auf einfache Weise zu überwachen. Dies ist beispielsweise dann von Vorteil, wenn die erfindungsgemässe Vorrichtung beziehungsweise das Verfahren zur Dosierung von Schmerzmedikamenten verwendet wird. So kann beispielsweise ein Arzt von Ferne die Schmerzsituation beurteilen und eine entsprechende Dosierung der Schmerzmedikamente verordnen.

In dem am meisten bevorzugten Verfahren zum Erfassen und Messen von Schmerzen eines Menschen wird mit Hilfe der Hand, und insbesondere durch Zudrücken der Hand, ein Druck auf eine elastische Aussenhülle 3a eines Hohlkörpers 3 ausgeübt und dabei ein Druck oder eine Kraft im Hohlkörper 3 gemessen, wobei der gemessene Druck oder die gemessene Kraft als Mass für den vom Menschen empfundene Schmerz verwendet wird.

In einem bevorzugten Verfahrensschritt wird vor jeder Messung der maximale Druck der Hand gemessen, um diese Maximalkraft Kₘₐₓ zu erfassen.

In einem weiteren, bevorzugten Verfahrensschritt wird vorgängig der Messung eine Kalibrierung durchgeführt, indem einem Patienten ein bestimmter Schmerz, vorzugsweise ein Wärmeschmerz, zugefügt wird, und indem danach der vom Patienten empfundene Schmerz mit der erfindungsgemässen Vorrichtung erfasst wird. Vorteilhafterweise werden dem Patienten eine Mehrzahl von Schmerzen unterschiedlicher Intensität zugeführt, beispielsweise nacheinander mit zunehmender Intensität, um die Messung zu kalibrieren. Vorteilhafterweise werden die beim Kalibrieren zugeführten bestimmten Schmerzen, und die gemessenen Werte der Schmerzen zudem in einer Datenbank gespeichert.

Es kann sich zudem als vorteilhaft erweisen die gemessenen Schmerzen mit in der Datenbank gespeicherten Schmerzen zu vergleichen. Die gemessenen Schmerzen können beispielsweise mit älteren Messungen desselben Patienten verglichen werden, oder auch mit den gemessenen Schmerzen von anderen Patienten.

In einem vorteilhaften Verfahren werden die Schmerzen während einem längeren Zeitintervall, insbesondere einer Stunde oder acht Stunden oder einem Tag gemessen, insbesondere in regelmässigen Zeitabständen.

In einem vorteilhaften Verfahren wird ein Schmerzschwellenwert vorgegeben und, wenn die gemessenen Schmerzen den Schmerzschwellenwert übersteigen, ein Signal erzeugt.

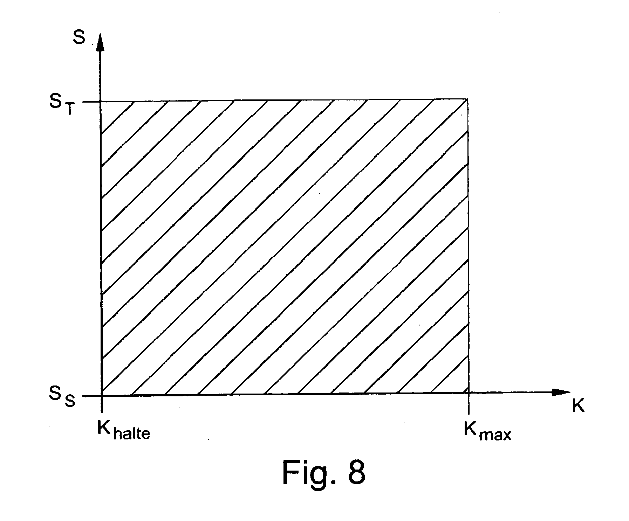

In einem vorteilhaften Verfahren wird die Scherzmessung, wie in Figur 8 dargestellt, derart geeicht, dass die Handkraft Kₕₐₗₜₑ, welche benötigt wird um die erfindungsgemässe Vorrichtung 1 gerade noch fest zu halten oder zu tragen, als Ausgangswert oder Nullwert eingestellt wird. Vorteilhafterweise werden die Schmerzwerte derart skaliert, dass der Schmerz-Schwellenwert Ss, bei welche ein Schmerz gerade noch spürbar ist oder gerade noch nicht spürbar ist, der Handkraft Kₕₐₗₜₑ zugeordnet wird. Es wird zudem die maximale Handkraft Kₘₐₓ gemessen, welche ein Patient zu erzeugen vermag. Dem maximal tolerierbaren Schmerzwert, dem sogenannten Schmerztoleranzwert S_{T}, wird die maximale Handkraft Kₘₐₓ zugeordnet. Die erfindungsgemässe Vorrichtung 1 wird somit vorteilhafterweise derart geeicht beziehungsweise kalibriert, dass die Messung in dem in Figur 8 schraffiert dargestellten Bereich durchgeführt wird.
Die erfindungsgemässe Vorrichtung 1 beziehungsweise das erfindungsgemässe Verfahren kann beispielsweise zur Dosierung von Schmerzmedikamenten verwendet werden, indem in einem ersten Schritt der gefühlte Schmerz gemessen wird, und indem in einem nachfolgenden Schritt das Schmerzmedikament abhängig von den gemessenen Schmerzen dosiert und dem Patienten verabreicht wird.

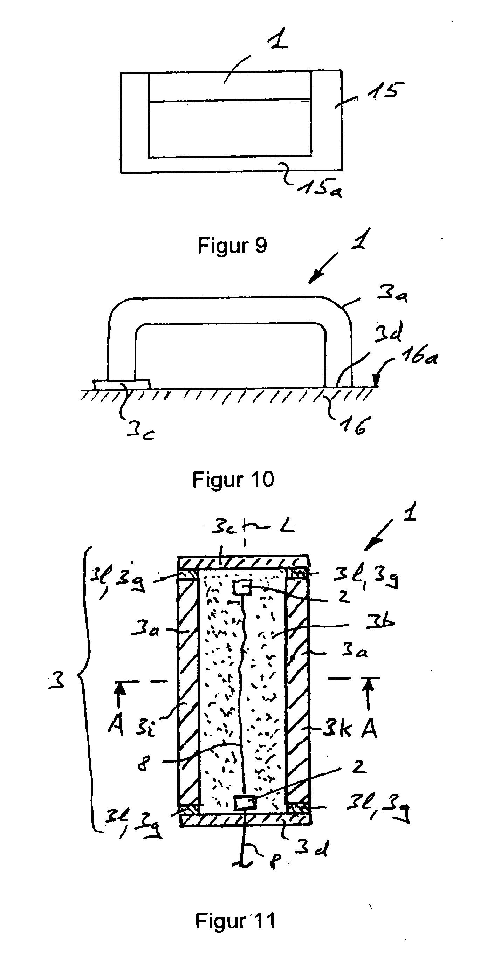

Figur 9 zeigt schematisch eine Seitenansicht eines weiteren Ausführungsbeispieles einer Vorrichtung 1 zum Erfassen und Messen von Schmerzen eines Menschen. Die Vorrichtung 1 ist an deren beiden Enden mit einer Haltevorrichtung 15 verbunden, wobei die Haltevorrichtung wie dargestellt vorzugsweise U-förmig ausgestaltet ist, wobei der Zwischenraum zwischen Vorrichtung 1 und Grundteil 15a derart ausgestaltet ist, dass zumindest die Finger einer Hand durchführbar sind, sodass die Vorrichtung 1 von einer Hand wie auch in Figur 2 dargestellt umschlossen werden kann. In einer vorteilhaften Ausgestaltung weist die Vorrichtung 1 wie dargestellt eine zylinderförmige beziehungsweise eine stangenförmige Aussenkontur auf. Das Grundteil 15a kann auch als ein Fuss ausgestaltet sein, der dazu dient die Vorrichtung 1 auf einen Untergrund zu stellen.

Figur 10 zeigt schematisch eine Seitenansicht eines weiteren Beispieles einer Vorrichtung 1 zum Erfassen und Messen von Schmerzen eines Menschen. Die Vorrichtung 1 weist eine u-förmig verlaufende Aussenhülle 3a auf, und weist an beiden Enden je ein Endteil 3c, 3d auf. Die Endteile 3c,3d können wie links mit dem Endteil 3c dargestellt als Fuss ausbildet sein, sodass die Vorrichtung 1 auf die Oberfläche 16a einer Unterlange 16 gestellt werden kann. Die Vorrichtung 1 ist derart ausgestaltet, dass in dem sich zwischen der Vorrichtung 1 und der Unterlage 16 ausbildenden Zwischenraum zumindest die Finger einer Hand durchführbar sind, sodass die Vorrichtung 1 von einer Hand, wie auch in Figur 2 dargestellt, umschlossen werden kann. Die Endteile 3c, 3d könnten auch, wie rechts mit dem Endteil 3c dargestellt, fest mit einem weiteren Gegenstand verbunden sein, zum Beispiel mit der Oberfläche 16a einer Wand 16. In einer vorteilhaften Ausgestaltung ist die Vorrichtung 1 batteriebetrieben ausgestaltet und weist eine kabellose Signalübertragung zu einer externen Sende- und Empfangsvorrichtung 12 auf. In einer vorteilhaften Variante weist die Vorrichtung 1, wie in Figur 10 dargestellt, hohlzylinderförmig verlaufende Teilabschnitte auf, welche über gekrümmte Teilabschnitte miteinander verbunden sind, sodass die Vorrichtung 1 als rohrförmiges Teil ausgestaltet ist.

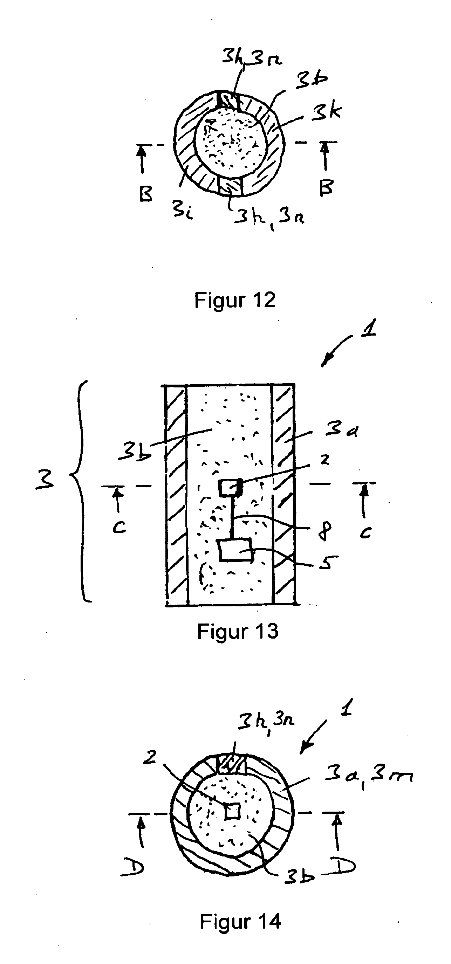

Die Figuren 11 und 12 zeigen ein weiteres Ausführungsbeispiel einer zylinder- beziehungsweise stabförmig ausgebildeten, in einer Längsrichtung L verlaufenden Vorrichtung 1 zum Erfassen und Messen von Schmerzen eines Menschen, wobei Figur 11 einen Längsschnitt entlang der Schnittlinie B-B und Figur 12 einen Querschnitt entlang der Schnittlinie A-A darstellt. Die Aussenhülle 3a umfasst zwei Halbschalen 3i, 3k, eine erste Halbschale 3i sowie eine zweite Halbschale 3k, welche einen Teil des Innenraums 3b begrenzen. Die Vorrichtung 1 umfasst zudem ein oberes sowie ein unteres Endteil 3c,3d, welche in der dargestellten Anordnung den Innenraums 3b oben und unten begrenzen. Die beiden Halbschalen 3i,3k sind derart ausgestaltet und in der Vorrichtung 1 angeordnet, dass über die beiden Halbschalen 3i,3k eine Kraft in den Innenraum 3b einleitbar ist, wobei die beiden Halbschalen 3i,3k eine geringere Elastizität aufweisen als das Medium im Innenraum 3b. In einer vorteilhaften Ausgestaltung weisen die beiden Halbschalen eine zumindest um einen Faktor 5 kleinere Elastizität auf als das Material 4a oder das Fluid 4b im Innenraum. In einer weiteren möglichen Ausgestaltung weisen die beiden Halbschalen eine viel grössere Elastizität auf, insbesondere eine um zumindest einen Faktor 5 oder zumindest einen Faktor 10 grössere Elastizität auf als das Material 4a oder das Fluid 4b im Innenraum. In einer bevorzugten Ausgestaltung sind die beiden Halbschalen 3i,3k gegeneinander verschiebbar angeordnet, vorzugsweise nur sehr geringfügig verschiebbar. Wie in Figur 12 dargestellt weisen die Halbschalen 3i, 3k einen gegenseitigen Spalt 3h auf, der entlang der ganzen Länge der Halbschalen 3i verläuft. Wie in Figur 11 dargestellt weist die Vorrichtung 1 zwischen den Halbschalen 3i, 3k und dem oberen Endteil 3c beziehungsweise dem unteren Endteil 3d ebenfalls einen Spalt 3g auf. In einer bevorzugten Ausgestaltung ist in dem in Richtung der Längsachse L verlaufenden Spalt 3h ein elastischer Streifen 3n aus nicht fliessfähigem Material wie zum Beispiel aus Kautschuk oder Gummi angeordnet. In einer bevorzugten Ausgestaltung ist zudem im ringförmigen Spalt 3g ebenfalls ein elastischer Ring 31 angeordnet, der vorzugsweise aus einem elastischen, nicht fliessfähigem Material wie zum Beispiel aus Kautschuk oder Gummi besteht. Bei dieser Ausgestaltung ist der Innenraum allseitig abgedichtet, sodass der Innenraum 3b auch mit einem flüssigen Fluid gefüllt sein könnte. Sofern der Innenraum 3b mit einem elastischen, nicht fliessfähigen Mittel gefüllt ist, so könnte der längsförmige Spalt 3h und/oder der ringförmige Spalt 3g auch mit demselben Mittel wie der Innenraum versehen sein. Im Innenraum 3b ist ein Druck- oder Kraftsensor 2 angeordnet um die über die Halbschalen 3i auf das Material im Innenraum 3b bewirkte Kraft zu messen. Im dargestellten Ausführungsbeispiel sind jeweils im Endabschnitt des Innenraums 3b, jeweils ein Sensor 2 angeordnet, wobei die Sensoren über ein elektrisch leitendes Kabel 8 miteinander und gegen aussen verbunden sind. Das sich im Innenraum 3b befindliche Material weist eine höhere Elastizität auf als die beiden Halbschalen 3i. Die beiden Halbschalen 3i können auch sehr starr ausgestaltet sein und beispielsweise aus einem Metall oder einem Hartkunststoff bestehen.

Figur 13 zeigt in einem Längsschnitt entlang der Schnittlinie D-D ein weiteres nicht erfindungsgemässes Beispiel einer Vorrichtung 1 zum Erfassen und Messen von Schmerzen. Im Vergleich zu der in Figur 11 dargestellten Vorrichtung 1 weist das in Figur 13 dargestellte Beispiel oben und unten kein Endteil 3c,3d auf, wobei der Innenraum 3b mit einem elastischen, nicht fliessfähigen Mittel wie Silikon, Kautschuk oder Gummi oder einem Gel gefüllt ist. Der Querschnitt dieses Beispiels könnte wie in Figur 12 oder 14 dargestellt ausgestaltet sein. Figur 14 zeigt in einem Querschnitt entlang der Schnittlinie C-C ein Ausführungsbeispiel bei welchem die Aussenhülle 3a ein im Wesentlichen c-förmigen ausgestaltetes federelastisches Teil 3m umfasst, das zwischen dessen Endabschnitten einen längsförmigen Spalt 3h ausbildet, welcher mit einem streifenförmigen elastischen Streifen 3n gefüllt ist, sodass der Innenraum 3b in Umfangsrichtung durch die c-förmige Schale 3m sowie den Streifen 3n umschlossen ist. In einer vorteilhaften Ausgestaltung weist das sich im Innenraum 3b befindliche Material eine höhere Elastizität auf als die c-förmige Schale 3m. In einem Beispiel könnte die c-förmige Schale 3m auch sehr starr ausgestaltet sein und beispielsweise aus einem Metall oder einem Hartkunststoff bestehen. Vorteilhafterweise ist der Streifen 3h elastischer ausgestaltet als die federelastische Schale 3m, sodass die Endabschnitte der federelastischen Schale 3m bei einer entsprechend angreifenden Kraft gegenseitig verschiebbar sind, sodass über den im Innenraum 3b angeordneten Druck- oder Kraftsensor 2 ein Signal messbar ist. Der Streifen 3h könnte auch mit demselben elastischen, nicht fliessfähigen Mittel wie der Innenraum 3b versehen sein. Im Innenraum 3b ist zudem eine Elektronikeinheit 5 angeordnet, welche über ein Kabel 8 mit dem Sensor 2 verbunden ist. Die Elektronikeinheit 5 könnte beispielsweise kabellos mit einer externen Vorrichtung 12 verbunden sein. Der hohlzylinderformige Hohlkörper 3 weist vorzugsweise einen Aussendurchmesser im Bereich zwischen 1cm und 5 cm auf.

Alle ausgeführten erfindungsgemässen Vorrichtungen 1 können eine Anzeigevorrichtung 7 umfassen. In einer weiteren vorteilhaften Ausführungsform könnte die beispielsweise in Figur 1 dargestellte Vorrichtung 1 derart ausgestaltet sein, dass die Aussenhülle 3a sowie der Innenraum 3b des Hohlkörpers 3 aus demselben Material besteht, einem elastischen, nicht fliesfähigen Material, wie beispielsweise Silikon, Kautschuk, Gummi oder ein Gel. In einer möglichen Variante besteht die Aussenhülle 3a sowie der Innenraum 3b aus demselben Material, welche vorzugsweise derart ausgestaltet sind, dass der Hohlköper 3 aus einem einzigen Teil besteht, mit darin oder daran anschliessend angeordnetem Druck- oder Kraftsensor 2.

## Patentansprüche

1. Vorrichtung (1) zum Erfassen und Messen von Schmerzen eines Menschen, umfassend einen Druck- oder Kraftsensor (2), umfassend einen Hohlkörper (3) mit einer Aussenhülle (3a) und einem Innenraum (3b), wobei die Aussenhülle (3a) den Innenraum (3b) zumindest teilweise begrenzt, sowie umfassend eine Elektronikeinheit (5) zum Erfassen des Signals des Druck- oder Kraftsensors (2), wobei die Aussenhülle (3a) des Hohlkörpers (3) derart ausgestaltet ist, dass diese von einer Hand zumindest teilweise umschliessbar ist, und wobei der Innenraum (3b) des Hohlkörpers (3) mit einem elastischen Material (4a) oder einem Fluid (4b) gefüllt ist, und wobei der Druck- oder Kraftsensor (2) derart angeordnet ist, dass der Druck des elastischen Materials (4a) oder des Fluides (4b) messbar ist, wobei die Aussenhülle (3a) hohlzylinderförmig ausgestaltet ist, die Aussenhülle (3a) mit einem oberen Endteil (3c) sowie einem unteren Endteil (3d) verbunden ist, das obere Endteil (3c), das untere Endteil (3d) sowie die Aussenhülle (3a) den Innenraum (3b) begrenzen, die Aussenhülle (3a) elastisch ausgestaltet ist, und das obere und untere Endteil (3c, 3d) starr ausgestaltet ist.

2. Vorrichtung (1) nach Anspruch 1, wobei das obere und/oder untere Endteil (3c, 3d) aus einem Kunststoff oder aus Metall gefertigt ist.

3. Vorrichtung (1) nach Anspruch 1 oder 2, wobei das obere und das untere Endteil (3c, 3d) gegenseitig fest miteinander verbunden sind.

4. Vorrichtung (1) nach einem der vorhergehenden Ansprüche, wobei das elastische Material (4a) eine geringere Elastizität aufweist als die Aussenhülle (3a).

5. Vorrichtung (1) nach Anspruch 1, wobei die Aussenhülle (3a) zwei Halbschalen (3i, 3k) umfasst, welche elastisch miteinander verbunden sind.

6. Vorrichtung (1) nach Anspruch 5, wobei die zwei Halbschalen (3i, 3k) eine zumindest um einen Faktor 5 grössere Elastizität aufweisen als das Material (4a) oder das Fluid (4b) im Innenraum, und die Halbschalen (3i, 3k) insbesondere aus einem Metall bestehen.

7. Vorrichtung nach einem der vorhergehenden Ansprüche, wobei der Hohlkörper (3) rohrförmig ausgestaltet ist und eine Längsachse (L) aufweist.

8. Vorrichtung nach einem der Ansprüche 1 bis 6, wobei am einen Ende des Hohlkörpers (3) der Druck- oder Kraftsensor (2) angeordnet ist.

9. Vorrichtung nach einem der Ansprüche 1 bis 7, wobei der rohrförmige Hohlkörper (3) in Längsrichtung (L) durch das obere Endteil (3c) und das untere Endteil (3d) begrenzt ist, und dass das obere Endteil (3c) eine rohrförmige Verlängerung (3e) aufweist, an deren einem Ende der Druck- oder Kraftsensor (2) angeordnet ist, und deren anderes Ende in den Innenraum (3b) des Hohlkörpers (3) mündet.

10. Vorrichtung nach Anspruch 8, wobei die rohrförmige Verlängerung (3e) konzentrisch zur Längsachse (L) verläuft.

11. Vorrichtung nach einem der Ansprüche 1 bis 9, wobei der Druck- oder Kraftsensor (2) im Inneren des Hohlkörpers (3) angeordnet ist.

12. Vorrichtung nach einem der vorhergehenden Anspruche, wobei der Hohlkörper (3) zumindest an denjenigen Stellen eine elastische Aussenhülle (3a) aufweist, welche als Auflage für eine Hand (13) bestimmt sind.

13. Vorrichtung nach einem der vorhergehenden Ansprüche, wobei diese einen Aktuator (10) zum Erzeugen von Schmerzen umfasst, wobei der Aktuator (10) insbesondere als eine Wärmeerzeugungsvorrichtung ausgestaltet ist, und/oder diese zusätzliche Sensoren (14) zum erfassen physiologischer Werte umfasst, insbesondere einen Sensor zum Erfassen der Hautleitfähigkeit, oder einen Sensor zur Erfassung des Pulses oder einen chemischen Sensor zum Erfassen biologischer Werte.

14. Vorrichtung nach einem der vorhergehenden Ansprüche, wobei die Elektronikeinheit (5) eine Auswertevorrichtung (5f) umfasst, welche es erlaubt aus den gemessenen Werten des Druck- oder Kraftsensors (2) einen Schmerzzustand eines Patienten zu berechnen.

15. Verwendung einer Vorrichtung nach einem der vorhergehenden Ansprüche zum Erfassen von Schmerzen eines Menschen.

## Claims

1. Device (1) for detecting and measuring pain of a human being, comprising a pressure or force sensor (2) comprising a hollow body (3) with an outer sheath (3a) and an interior (3b), wherein the outer sheath (3a) at least partially bounds the interior (3b), and comprising an electronic unit (5) for detecting the signal of the pressure or force sensor (2), wherein the outer sheath (3a) of the hollow body (3) is configured in such a way that it can be at least partially enclosed by a hand, and wherein the interior (3b) of the hollow body (3) is filled with an elastic material (4a) or a fluid (4b) and wherein the pressure or force sensor (2) is arranged in such a way that the pressure of the elastic material (4a) or of the fluid (4b) can be measured, wherein the outer sheath (3a) is configured in the form of a hollow cylinder, the outer sheath (3a) is connected to an upper end part (3c) and a lower end part (3d), the upper end part (3c), the lower end part (3d) and the outer sheath (3a) bound the interior (3b), the outer sheath (3a) is embodied in an elastic fashion, and the upper and lower end parts (3c, 3d) are embodied in a rigid fashion.

2. Device (1) according to Claim 1, wherein the upper and/or lower end parts (3c, 3d) are fabricated from a plastic or a metal.

3. Device (1) according to Claim 1 or 2, wherein the upper and the lower end parts (3c, 3d) are fixedly connected to one another.

4. Device (1) according to one of the preceding claims, wherein the elastic material (4a) has lower elasticity than the outer sheath (3a).

5. Device (1) according to Claim 1, wherein the outer sheath (3a) comprises two half-shells (3i, 3k) which are elastically connected to one another.

6. Device (1) according to Claim 5, wherein the two half-shells (3i, 3k) have elasticity which is greater at least by a factor of 5 than the material (4a) or the fluid (4b) in the interior, and the half-shells (3i, 3k) are composed in particular of a metal.

7. Device according to one of the preceding claims, wherein the hollow body (3) is embodied in a tubular fashion and has a longitudinal axis (L).

8. Device according to one of Claims 1 to 6, wherein the pressure or force sensor (2) is arranged at one of the ends of the hollow body (3).

9. Device according to one of Claims 1 to 7, wherein the tubular hollow body (3) is bounded in the longitudinal direction (L) by the upper end part (3c) and the lower end part (3d), and in that the upper end part (3c) has a tubular extension (3e) at one end of which the pressure or force sensor (2) is arranged and the other end of which leads into the interior (3b) of the hollow body (3).

10. Device according to Claim 8, wherein the tubular extension (3e) runs concentrically with respect to the longitudinal axis (L).

11. Device according to one of Claims 1 to 9, wherein the pressure or force sensor (2) is arranged in the interior of the hollow body (3).

12. Device according to one of the preceding claims, wherein the hollow body (3) has an elastic outer sheath (3a) at least at those locations which are intended as a support for a hand (13).

13. Device according to one of the preceding claims, wherein said device comprises an actuator (10) for generating pain, wherein the actuator (10) is configured, in particular, as a heat-generating device, and/or said device comprises additional sensors (14) for detecting physiological values, in particular a sensor for detecting the conductivity of the skin, or a sensor for detecting the pulse or a chemical sensor for detecting biological values.

14. Device according to one of the preceding claims, wherein the electronic unit (5) comprises an evaluation device (5f) which permits a pain level of a patient to be calculated from the measured values of the pressure or force sensor (2).

15. Use of a device according to one of the preceding claims for detecting pain of a human being.

## Revendications

1. Dispositif (1) pour détecter et mesurer la douleur d'une personne, comprenant un capteur de pression ou de force (2), comprenant un corps creux (3) avec une enveloppe extérieure (3a) et un espace interne (3b), l'enveloppe extérieure (3a) limitant au moins en partie l'espace interne (3b), et comprenant une unité électronique (5) pour détecter le signal du capteur de pression ou de force (2), l'enveloppe extérieure (3a) du corps creux (3) étant configurée de telle sorte qu'elle puisse être entourée au moins en partie par une main et l'espace interne (3b) du corps creux (3) étant rempli d'un matériau élastique (4a) ou d'un fluide (4b), et le capteur de pression ou de force (2) étant disposé de telle sorte que la pression du matériau élastique (4a) ou du fluide (4b) puisse être mesurée, l'enveloppe extérieure (3a) étant configurée sous forme cylindrique creuse, l'enveloppe extérieure (3a) étant connectée à une partie d'extrémité supérieure (3c) et à une partie d'extrémité inférieure (3d), la partie d'extrémité supérieure (3c), la partie d'extrémité inférieure (3d) ainsi que l'enveloppe extérieure (3a) limitant l'espace interne (3b), l'enveloppe extérieure (3a) étant configurée sous forme élastique, et la partie d'extrémité supérieure et la partie d'extrémité inférieure (3c, 3d) étant configurées sous forme rigide.

2. Dispositif (1) selon la revendication 1, dans lequel la partie d'extrémité supérieure et/ou la partie d'extrémité inférieure (3c, 3d) sont fabriquées en un plastique ou en métal.

3. Dispositif (1) selon la revendication 1 ou 2, dans lequel la partie d'extrémité supérieure et la partie d'extrémité inférieure (3c, 3d) sont connectées fixement l'une à l'autre.

4. Dispositif (1) selon l'une quelconque des revendications précédentes, dans lequel le matériau élastique (4a) présente une plus faible élasticité que l'enveloppe extérieure (3a).

5. Dispositif (1) selon la revendication 1, dans lequel l'enveloppe extérieure (3a) présente deux demi-coques (3i, 3k) qui sont connectées élastiquement l'une à l'autre.

6. Dispositif (1) selon la revendication 5, dans lequel les deux demi-coques (3i, 3k) présentent une élasticité supérieure d'au moins un facteur 5 à celle du matériau (4a) ou du fluide (4b) dans l'espace interne, et les demi-coques (3i, 3k) se composent notamment d'un métal.

7. Dispositif selon l'une quelconque des revendications précédentes, dans lequel le corps creux (3) est configuré sous forme tubulaire et présente un axe longitudinal (L).

8. Dispositif selon l'une quelconque des revendications 1 à 6, dans lequel le capteur de pression ou de force (2) est disposé à une extrémité du corps creux (3).

9. Dispositif selon l'une quelconque des revendications 1 à 7, dans lequel le corps creux tubulaire (3) est limité dans la direction longitudinale (L) par la partie d'extrémité supérieure (3c) et la partie d'extrémité inférieure (3d), et la partie d'extrémité supérieure (3c) présente un prolongement tubulaire (3e), à l'une des extrémités duquel est disposé le capteur de pression ou de force (2), et dont l'autre extrémité débouche dans l'espace interne (3b) du corps creux (3).

10. Dispositif selon la revendication 8, dans lequel le prolongement tubulaire (3e) s'étend concentriquement à l'axe longitudinal (L).

11. Dispositif selon l'une quelconque des revendications 1 à 9, dans lequel le capteur de pression ou de force (2) est disposé à l'intérieur du corps creux (3).

12. Dispositif selon l'une quelconque des revendications précédentes, dans lequel le corps creux (3) perce au moins une enveloppe extérieure élastique (3a) au moins aux endroits qui sont prévus en tant qu'appui pour une main (13).

13. Dispositif selon l'une quelconque des revendications précédentes, celui-ci comprenant un actionneur (10) pour générer de la douleur, l'actionneur (10) étant configuré en particulier sous forme de dispositif de génération de chaleur et/ou celui-ci comprenant des capteurs supplémentaires (14) pour détecter des valeurs physiologiques, en particulier un capteur pour détecter la conductibilité de la peau, ou un capteur pour détecter le pouls ou un capteur chimique pour détecter des valeurs biologiques.

14. Dispositif selon l'une quelconque des revendications précédentes, dans lequel l'unité électronique (5) comprend un dispositif d'analyse (5f) qui permet de calculer, à partir des valeurs mesurées du capteur de pression ou de force (2), un état de douleur d'un patient.

15. Utilisation d'un dispositif selon l'une quelconque des revendications précédentes, pour détecter la douleur d'une personne.
